# EUROPEAN PATENT APPLICATION

(11) **EP 2 732 810 A1**
(43) Date of publication of application: **21.05.2014**
(21) Application number: 11869405.8
(22) Date of filing: 27.10.2011
(51) Int. Cl.: A61K 9/16, A61K 9/20, C07D 495/04, A61K 31/4365

(54) **SPHERICAL PARTICLES OF CLOPIDOGREL BISULFATE, PHARMACEUTICAL COMPOSITION INCLUDING SAME, AND METHOD FOR MANUFACTURING SAME**

(30) Priority: 12.07.2011 KR 20110069039
(71) Applicant: Samjin Pharmaceutical Co., Ltd., Seoul 121-836 (KR); Astech Co., Ltd., Gyeonggi-do 441-813 (KR)
(72) Inventor: CHO, Eui Hwan, Seoul 140-030 (KR); SHIN, Hee Jong, Bucheon-si Gyeonggi-do 420-761 (KR); SONG, Woo Heon, Suwon-si Gyeonggi-do 441-704 (KR); LEE, Sun Hwan, Hwaseong-si Gyeonggi-do 445-788 (KR); YOON, Jong Bae, Suwon-si Gyeonggi-do 440-300 (KR); PARK, Jong Sung, Seoul 143-210 (KR)
(74) Representative: González López-Menchero, Álvaro Luis
(86) International application number: PCT/KR2011/008071
(87) International publication number: WO 2013/008981

(57) **Abstract**

The present invention relates to spherical particles of clopidogrel bisulfate and a pharmaceutical composition containing the same. The inventive spherical particles can be used for preparing a tablet having sufficient hardness through direct compression, by improving unformulable properties of clopidogrel bisulfate such as compressibility, flowability and strong surface electrostatic charges, reduce a problem in compressing tablets such as weight variation, sticking, etc., and the risk of form conversion, and improve physiochemical stability. Therefore, the spherical particles of the present invention may be used as therapeutics for arteriosclerosis, stroke, myocardial infarction and atherosclerosis.

## Description

### FIELD OF THE INVENTION

The present invention relates to spherical particles of clopidogrel bisulfate and a method for preparing the same, and more particularly to spherical particles of clopidogrel bisulfate having a particle size (d0.1) of 30 µm or more and a particle size (d0.5) of 50 to 200 µm, a pharmaceutical composition or a tablet containing the same, and a method for preparing the same.

### BACKGROUND OF THE INVENTION

Clopidogrel, whose chemical name is methyl (+)-(S)-α-(o-chlorophenyl)-6,7-dihydrothieno[3,2-c]pyridin-5(4H)-acetate, is a representative thienopyridine class antiplatelet agent. Clopidogrel is an inhibitor of platelet aggregation which acts by inhibiting the binding of adenosine diphosphate to its receptor. The antiplatelet activity of clopidogrel makes it an effective drug for arteriosclerosis, stroke, myocardial infarction, atherosclerosis and the like. Korean Patent No. 10-24333 discloses clopidogrel and a method for preparing the same, and Korean Patent No. 10-103094 describes a method for preparing a dextro-rotatory enantiomer of clopidogrel, which also exemplifies, as a salt thereof, hydrochloride, bisulfate, bromate and taurocholate, preferably clopidogrel bisulfate. Clopidogrel bisulfate is marketed under the trade name "Plavix®" (Sanofi-Aventis).

Clopidogrel bisulfate was first disclosed in EP Patent No. 281,459, and it has been reported that clopidogrel bisulfate can exist in different crystalline forms such as Forms I, II, III, IV, V and VI. Among them, only Form I and Form II are used in pharmaceutical applications (U.S. Patent No. 6,504,030).

It is known that the crystalline Form I of clopidogrel bisulfate (monoclinic) is more dense (Form I: 1.505 g/cm², Form II: 1.462 g/cm²) and soluble than Form II (orthorhombic) (see U.S. Patent No. 6,429,210). However, clopidogrel bisulfate crystalline Form I is often converted to more stable Form II, due to relative low thermodynamic stability. The conversion from Form I to Form II gradually increases over the course of time by solvent, heat, light, or other various external environmental conditions.

Methods for preparing of crystalline Forms I and II of clopidogrel bisulfate have been reported by a number of documents (see Korean Patent No. 10-0511238; Korean Patent Laid-open Publication No. 10-2007-0106674; Korean Patent Laid-open Publication No. 10-2006-0026119; Korean Patent Laid-open Publication No. 10-2010-0128051; and Korean Patent Laid-open Publication No. 10-2008-0060420).

Further, a method was disclosed which comprises dissolving clopidogrel free base in a variety of organic solvents and adding a strong sulfuric acid thereto in Korean Patent No. 10-0511238, Korean Patent Laid-open Publication No. 10-2007-0106674, Korean Patent Laid-open Publication No. 10-2006-0026119, Korean Patent Laid-open Publication No. 10-2010-0128051, Korean Patent Laid-open Publication No. 10-2008-0060420 etc. However, such method has a problem resulting in fine powder-type of particles instead of crystal particles having rod-shape. In addition, the powder-type particles contain a substantial amount of mother liquid with sulfuric acid in spite of filtering or washing step, and the remaining sulfuric acid oxidizes clopidogrel on and after drying step, giving rise to discoloration and severe unpleasant odors.

It is well known to a person skilled in the art that the crystalline form and size of particles suitable for formulation is critical besides its homogeneity, so as to formulate main ingredients into a tablet, a powder, a capsule, etc. However, above-mentioned documents merely focus on preparing crystalline Form I or II as a pure crystal form, and do not teach crystal particle form or size as well as methods for preparing the crystal particles according to its form or size.

As a formulation containing clopidogrel bisulfate as an active ingredient, a tablet is widely employed by virtue of many advantages including stability, homogeneity of main ingredients, modifiability of appearance as well as prevention from degradation of main ingredients by blocking moisture and light via surface coating.

Tablets are prepared by three general methods: wet granulation, dry granulation and direct compression.

Wet granulation is widely used, which comprises mixing main ingredients and additives such as excipients, binding agents, disintegrating agents and the like, adding a solvent or a binder solution in a solvent to make granules, drying and assembling the granules, mixing it with a lubricating agent, and compressing the granules. However, when clopidogrel bisulfate is formulated into a tablet by using wet granulation, it is difficult to maintain its homogeneity because of conversion from crystalline Form I to Form II on granulation with a solvent, as well as serious physicochemical instability such as discoloration, increased impurities, reduced ingredients, etc.

Dry granulation comprises pulverizing and assembling a slug or sheet, which is obtained through dry-combination using a low-speed tablet press machine or a roller compressor, mixing with a lubricant agent, and compressing the mixture. However, when clopidogrel is formulated into a tablet by using dry granulation, there is an increased risk of impurities resulted from elevated temperature due to the use of a low-speed tablet press machine or a roller compressor.

Direct compression comprises mixing main ingredients with an excipient, a binding agent, a disintegrating agent, a lubricating agent and the like, and immediately compressing the mixture. The process is simple, has no risk of form conversion, and ensures stability. However, if the weight ratio of main ingredients exceeds 20%, direct compression method cannot be easily applied, though it compensates the physical properties of main ingredients using an excipient with good compressibility and flowability, specially designed for direct compression. Meanwhile, as the weight ratio of the main ingredient is 30% or higher for clopidogrel bisulfate tablet, additional binding agents are required to obtain a tablet with sufficient hardness, and if fine powder particles of high surface electrostatic charges and poor flowability are contained, it occurs serious weight variation and a problem in compressing tablets such as sticking onto a punch, which is not suitable for mass production.

Accordingly, many researches have been focused on developing a method suitable for mass production. As an example, Korean Patent Laid-open Publication No. 10-2008-0098964 discloses a clopidogrel bisulfate composition prepared by direct compression using, as a lubricating agent, a mixture of vegetable hardened oil, talc and silicon dioxide. However, for the mass production of clopidogrel bisulfate fine powder particles, there remains a problem in compressing tablets, e.g., weight variation, sticking and the others, and vegetable oil with low-melting point used as a lubricating agent tends to easily melt by heat generated from long-term, high-speed tableting, thereby adversely affecting the stability of clopidogrel bisulfate and acting as a medium for form conversion.

Additionally, Korean Patent No. 10-0809903 discloses a method for coating clopidogrel with hydroxypropylcellulose or hydroxypropylmethylcellulose. However, the process is so complicated and not suitable for mass production, gives rise to conversion of crystalline forms and decreased chemical stability on using solvents, and makes the size of the tablet too large due to the use of a substantial amount of coating agents.

Surface electrostatic charges and adherence of clopidogrel bisulfate occur in fine particles, i.e., fine particles of 30 µm or smaller. The smaller particles have a large contact area with air to easily absorb moisture, and are light and tend to adhere to each other even by a low electrostatic, thereby producing a problem in compressing tablets. Further, even if particles exceeds 30 µm, particles with coarse surface may hinder flowability, and excessively large particles, if present in a large amount, may cause weight variation and difficulty in mixing and sieving, as well as produce fine particles from abrasion during process, resulting in a problem in compressing tablets.

Therefore, in order to completely solve these problems, it is preferred to prepare a formulation to contain a trace of fine particles not to cause a problem in compressing tablets, and to make all particles into smooth, spherical particles having a size of 30 µm or more. However, clopidogrel particle forms, sizes, and preparation methods have not yet been known until now.

There are a number of factors determining shapes of crystalline particles during crystallization process. Exemplary factors include: (a) intrinsic solid properties of clopidogrel bisulfate (e.g., lattice structure and form, strength, hardness, abrasion resistance, etc.); (b) characteristics of solvents for crystallization (solubility, density, temperature, viscosity, degree of supersaturation, concentration, ionic strength, etc.); and (c) conditions for crystallization (e.g., stirring method, stirring strength, input time, input ratio, input point, seed crystal, etc.). The diversity of these factors is the obstacles to determination of optimal particle form.

The present inventors have prepared spherical agglomerates and spherical spherulites of clopidogrel bisulfate having a particular particle distribution by optimizing above factors, and have found that the particles can effectively solve previous problems.

### SUMMARY OF THE INVENTION

It is an object of the present invention to provide spherical particles of clopidogrel bisulfate having characteristics suitable for preparing a tablet by direct compression.

It is other object of the present invention to provide a pharmaceutical composition containing the spherical particles of clopidogrel bisulfate and a method for preparing the same.

It is another object of the present invention to provide a tablet having an improved physicochemical stability, prepared from such pharmaceutical composition, and a preparation method thereof.

In accordance with one aspect of the present invention, there is provided spherical particles of clopidogrel bisulfate having a particle size (d0.1) of 30 µm or more and a particle size (d0.5) of 50 to 200 µm.

In accordance with other aspect of the present invention, there is provided a pharmaceutical composition comprising the spherical particles of clopidogrel bisulfate; a pharmaceutically acceptable excipient for direct compression; low-substituted hydroxypropylcellulose as a disintegrating agent; colloidal silicon dioxide as a fludizing agent; and sodium stearyl fumarate as a lubricating agent.

In accordance with another aspect of the present invention, there is provided a tablet prepared from the pharmaceutical composition by direct compression.

In accordance with another aspect of the present invention, there is provided a method for preparing a tablet containing spherical particles of clopidogrel bisulfate, comprising the steps of: 1) mixing the spherical particles of clopidogrel bisulfate; a pharmaceutically acceptable excipient for direct compression; low-substituted hydroxypropylcellulose as a disintegrating agent; colloidal silicon dioxide as a fludizing agent; and sodium stearyl fumarate as a lubricating agent; and 2) subjecting the mixture to direct compression without adding a binding agent, to form a tablet.

The inventive spherical particles can be used for preparing a tablet having sufficient hardness through direct compression, by improving unformulable properties of clopidogrel bisulfate such as compressibility, flowability and strong surface electrostatic charges, reduce a problem in compressing tablets such as weight variation, sticking, etc., and the risk of form conversion, and improve physiochemical stability. Therefore, the spherical particles of the present invention may be used as therapeutics for arteriosclerosis, stroke, myocardial infarction and atherosclerosis.

### BRIEF DESCRIPTION OF DRAWINGS

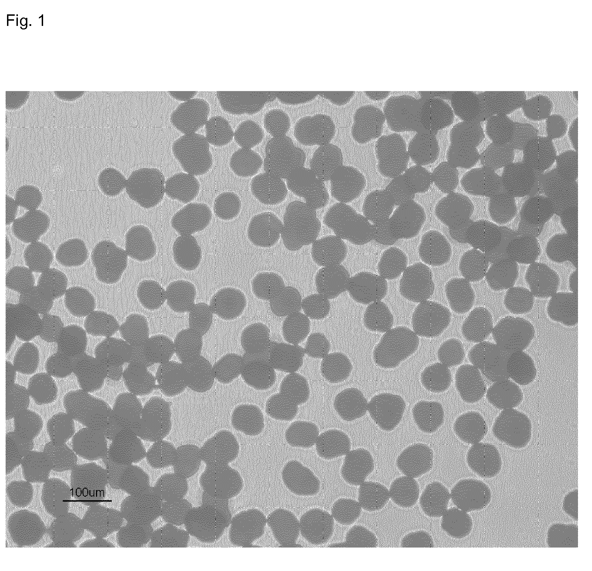

Fig. 1 is a microscope photograph of the spherical particles of clopidogrel bisulfate Form I, obtained in Example 1.

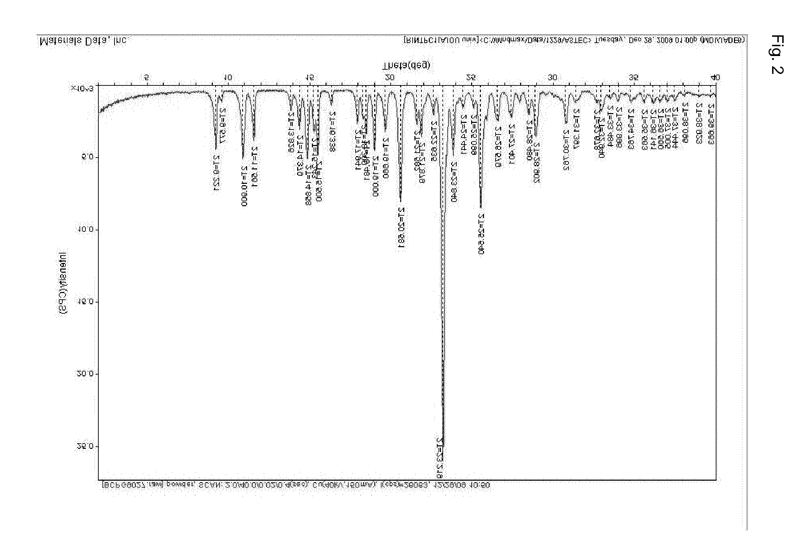

Fig. 2 is an X-ray diffraction pattern of the spherical particles of clopidogrel bisulfate Form I, obtained in Example 1.

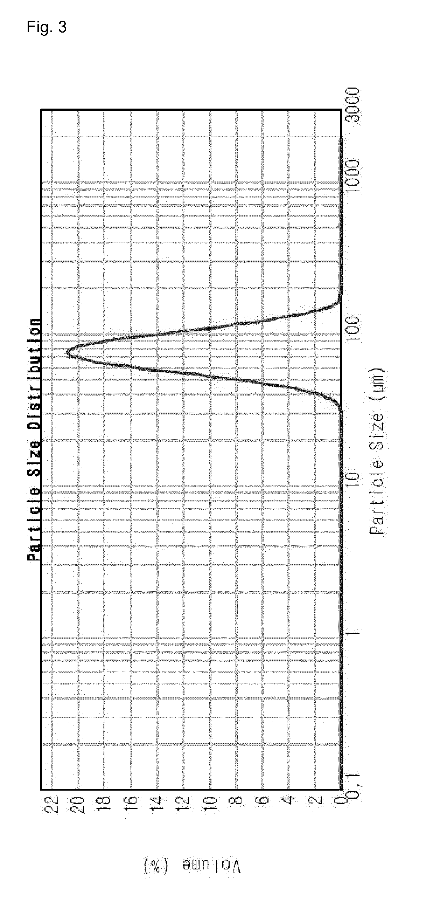

Fig. 3 is a particle size distribution of the spherical particles of clopidogrel bisulfate Form I, obtained in Example 1.

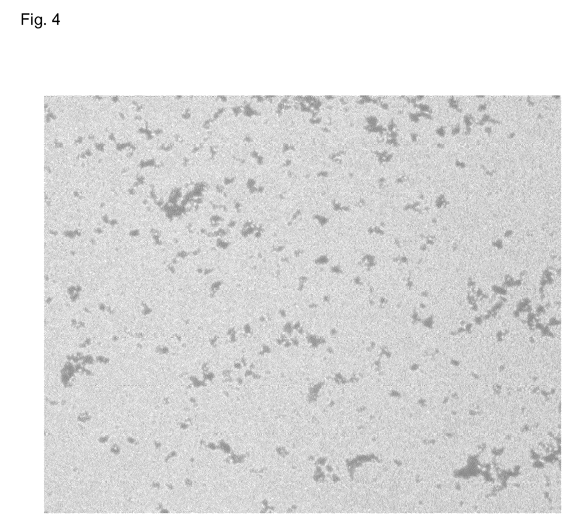

Fig. 4 is a microscope photograph of the powder crystals of clopidogrel bisulfate Form I, obtained in Comparative Example 1.

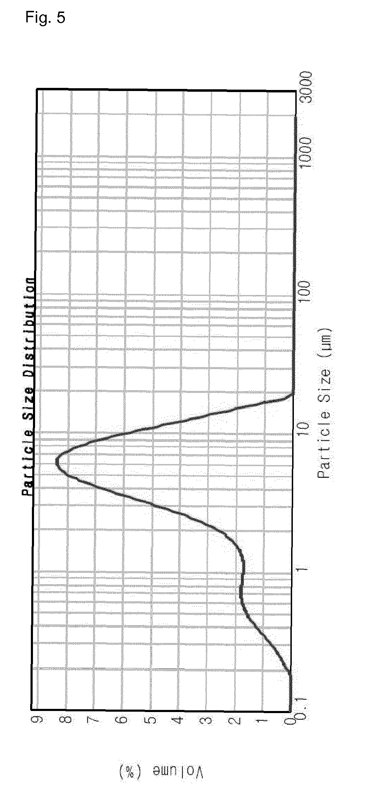

Fig. 5 is a particle size distribution of the powder crystals of clopidogrel bisulfate form I, obtained in Comparative Example 1.

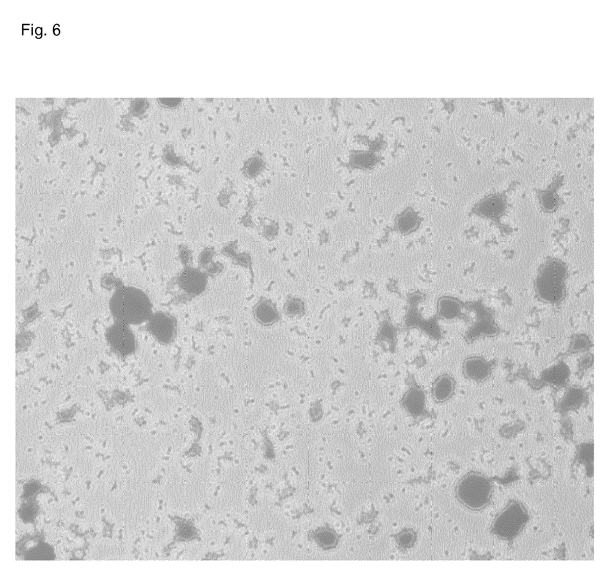

Fig. 6 is a microscope photograph of the powder crystals of clopidogrel bisulfate form I, obtained in Comparative Example 2.

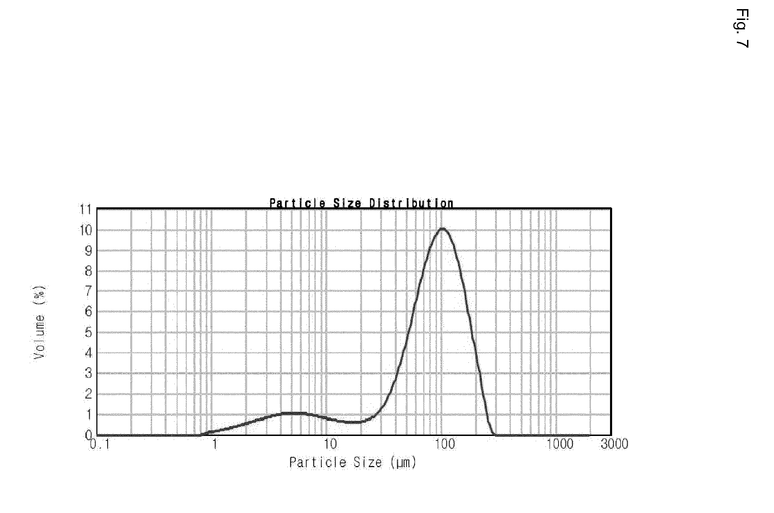

Fig. 7 is a particle size distribution of the powder crystals of clopidogrel bisulfate form I, obtained in Comparative Example 2.

### DETAILED DESCRIPTION OF THE INVENTION

Hereinafter, the present invention is described in detail.

The present invention provides spherical particles of clopidogrel bisulfate having a particle size (d0.1) of 30 µm or more and a particle size (d0.5) of 50 to 200 µm.

The clopidogrel used herein is well known as an antiplatelet agent in the art, which is effective in arteriosclerosis, stroke, myocardial infarction, atherosclerosis and the like.

The clopidogrel bisulfate and a method for preparing the same are well known in the art. The clopidogrel bisulfate may exist in crystalline Form I, Form II, or a mixture thereof.

The spherical particles of clopidogrel bisulfate according to the present invention are characterized by having a particle size (d0.1) of 30 µm or more and a particle size (d0.5) of 50 to 200 µm.The term "particle size (d0.1 )" as used herein refers to a particle diameter at a point which the cumulative frequency of volumetric distribution starting from particles with smallest diameter reaches 10%, and the term "particle size (d0.5)" as used herein refers to a particle diameter at a point which the cumulative frequency of volumetric distribution starting from particles with smallest diameter reaches 50%. That is, the spherical particles of clopidogrel bisulfate in accordance with the present invention has a particle diameter of 30 µm or more at a point which the cumulative frequency of volumetric distribution starting from particles with smallest diameter reaches 10%, and has a particle diameter of 50 to 200 µm at a point which the cumulative frequency of volumetric distribution starting from particles with smallest diameter reaches 50%. More preferably, the spherical particles of clopidogrel bisulfate have a particle size (d0.1) of 50 µm or more and a particle size (d0.5) of 60 to 100 µm.

When the particle size (d0.1) is less than 30 µm, the compressibility and flowability of the particles are poor, the surface electrostatic charges and adherence become higher, causing a problem in compressing tablets such as weight variation, sticking, etc., and if the particles are not smooth, the flowability is deteriorated on compressing tablets.

Further, when particle size (d0.5) is more than 200 µm, weight variation on compressing tablets may occur, processes such as mixing and sieving may be difficult to be performed, and fine particles produced by abrasion cause a problem in compressing tablets.

In addition, the present invention provides a method for preparing spherical particles of clopidogrel bisulfate. The present inventors have invented a method for preparing spherical agglomerates by piling up particles around a seed particle and a method for preparing spherical spherulites by polycrystallizing particles radially initiated from starting particle nucleus. Furthermore, the present inventors have found that spherical spherulites of clopidogrel bisulfate are mainly crystalline Form II, and that spherical agglomerates of clopidogrel bisulfate are mainly crystalline Form I. The method for preparing spherical particles of the present invention comprises the following steps:
(i) dissolving clopidogrel free base in 2-butanol;
(ii) adding clopidogrel seed crystals to the solution;
(iii) adding sulfuric acid diluted with cyclohexane dropwise to the solution;
(iv) stirring the mixed solution to prepare spherical particles; and
(v) filtering, washing and drying the particles to obtain spherical particles.

In the method, the clopidogrel free base used as a starting material may be obtained by any process known in the art, or commercially available. For instance, Korean Patent No. 10-0511238 describes that dextro-rotatory clopidogrel free base can be obtained by dissolving clopidogrel (1 R)-(-)-camphor-10-sulfonate in dichloromethane, adding an aqueous solution of potassium carbonate, stirring the mixture, and concentrating the organic phase under vacuum.

The amount of clopidogrel free base used as a starting material in the method according to the present invention may vary depending upon a solvent chosen, preferably about 5 to 20% by weight.

Meanwhile, the step (ii) and step (iii) in the inventive method may be carried out at a temperature of 0~40°C, preferably a temperature of 26-28°C, and the clopidogrel seed crystals in step (ii) may be added in an amount of 5 to 20% by weight.

Further, the amount of sulfuric acid used in step (iii) in the inventive method may vary depending upon a solvent chosen, preferably 2 to 50 % by weight.

Furthermore, the stirring in step (iv) in the inventive method may be carried out at a temperature of 0 to 40°C, preferably at a temperature of 17-19°C, for 30 min to 24 hours, and the drying in step (v) may be preferably carried out a temperature of 70~80°C under vacuum.

The spherical particles prepared as above may improve unformulable properties of clopidogrel bisulfate such as poor compressibility, flowability and strong surface electrostatic charges. In addition, when formulated into a tablet by direct compression, the spherical particles provides a sufficient hardness to a tablet without an additional binding agent, reduce a problem in compressing tablets such as weight variation, sticking, etc., and a risk of form conversion, and improve physiochemical stability of tablets. Therefore, the spherical particles of the present invention may be used as an effective ingredient in therapeutics for arteriosclerosis, stroke, myocardial infarction and atherosclerosis.

The present invention provides a pharmaceutical composition comprising the spherical particles of clopidogrel bisulfate. The spherical particles of clopidogrel bisulfate are contained in an amount of 30 to 50% by weight based on the total weight of the tablet. Meanwhile, the pharmaceutical composition of the present invention may further comprise a pharmaceutically acceptable excipient for direct compression; a disintegrating agent; a fludizing agent; and a lubricating agent, in addition to the spherical particles of clopidogrel bisulfate. The pharmaceutically acceptable excipient for direct compression may be any one selected from the group consisting of lactose hydrate, anhydrous lactose, mannitol, microcrystalline cellulose, silicified microcrystalline cellulose and a mixture thereof, and it may be contained in an amount of 30 to 50% by weight based on the total weight of the tablet. The disintegrating agent may be preferably low-substituted hydroxypropylcellulose, and it may be contained in an amount of 5 to 15% by weight based on the total weight of the tablet. The fludizing agent may be preferably colloidal silicon dioxide, and it may be contained in an amount of 0.5 to 2% by weight based on the total weight of the tablet. The lubricating agent may be preferably sodium stearyl fumarate, and it may be contained in an amount of 1 to 4% by weight based on the total weight of the tablet. When a conventional lubricating agent, e.g., magnesium stearate or zinc stearate, is used in the pharmaceutical composition, it may deteriorate physical/chemical stability of clopidogrel bisulfate due to the reactivity of metal ions, and when vegetable oil is used in the pharmaceutical composition, it may cause sticking due to low meting-point and, if melted by heat generated from long-term, high-speed tableting, may act as a solvent to lower stability of clopidogrel bisulfate and as a medium for form conversion.

Further, the pharmaceutical composition comprising the spherical particles of the present invention is characterized by comprising no additional binding agent. Typically, since it is difficult to prepare a tablet with sufficient hardness when a lot amount of active ingredients is present in a pharmaceutical composition, the composition should further contain a binding agent such as copolyvidone. However, such binding agent may interact with clopidogrel bisulfate to bring about serious problems such as discoloration and increase in impurities. As the spherical particles of clopidogrel bisulfate according to the present invention have a superior compressibility, they can be formulated into a tablet with sufficient hardness in high-speed tableting, even with no additional binding agent.

Accordingly, the present invention provides a tablet prepared from the pharmaceutical composition by direct compression.

Moreover, the present invention provides a method for preparing a tablet containing spherical particles of clopidogrel bisulfate. The tablet may be an uncoated tablet or a coated tablet. In an aspect of the present invention, the tablet of the present invention is prepared by: 1) mixing the spherical particles of clopidogrel bisulfate; a pharmaceutically acceptable excipient for direct compression; low-substituted hydroxypropylcellulose as a disintegrating agent; colloidal silicon dioxide as a fludizing agent; and sodium stearyl fumarate as a lubricating agent; and 2) subjecting the mixture to direct compression without adding a binding agent, to form a tablet.

Direct compression method is preferable for preparing a tablet containing clopidogrel bisulfate. The spherical particles of the clopidogrel bisulfate have an improved flowability and surface electrostatic charges, thereby significantly reducing the risk of a problem in compressing tablets, and have a superior compressibility to enhance production efficiency. In addition, they reduce significantly the risk of form conversion of clopidogrel bisulfate, and improve physiochemical stability.

Meanwhile, the tablet may be coated with an appropriate coating agent, if needed, e.g., for giving particular colors. The spherical particles of clopidogrel bisulfate according to the present invention may be orally administered to a mammal, at a dosage of 1 to 1,000 mg/kg body weight, preferably 25 to 250 mg/kg body weight, in one or more times daily, as a single dose or divided dose.

Hereinafter, the present invention is described in more detail. The following Examples are given for the purpose of illustration only, and are not intended to limit the scope of the invention.

### Example 1: Preparation of spherical particles of clopidogrel bisulfate (crystalline Form I)

### <1-1> Preparation of clopidogrel free base

554 g (1.0 mol) of clopidogrel (1R)-(-)-camphor-10-sulfonate was dissolved in 300 mL of dichloromethane, and a solution of potassium carbonate (138.3 g) in water (300 mL) was added thereto. The mixture was stirred for 30 min, dried over anhydrous sodium sulfate, and concentrated under reduced pressure to obtain clopidogrel free base as oil (yield: 99%).

### <1-2> Preparation of spherical particles of clopidogrel bisulfate (crystalline Form I)

200 g of clopidogrel free base was dissolved in 2,000 mL of 2-butanol, followed by maintaining at 26 to 28°C. Then, crystalline Form I as a seed crystal (5-20% relative to the amount of clopidogrel free base) was added thereto. Subsequently, a solution of 62.3 g of sulfuric acid (98%) in 400 mL of cyclohexane was added dropwise thereto for 8 to 10 hours, while cooling the solution to 0.5 to 2°C. After addition, the mixture was adjusted to a temperature of 17 to 19°C. Thirty minutes to one hour after addition, the mixture was filtered to obtain spherical crystals. The filtered crystals were dried at 70-80°C for 24 hours to obtain 258 g of spherical particles of crystalline Form I (yield 95%).

### Comparative Example 1: Preparation of powder-type particles of clopidogrel bisulfate (crystalline Form I)

200 g of clopidogrel free base obtained in Example <1-1> was dissolved in 2,000 mL of ethyl acetate, followed by maintaining at 26 to 28°C. Then, a solution of 62.3 g of sulfuric acid (98%) in 400 mL of cyclohexane was added dropwise thereto for 1 hour. The mixture was vigorously stirred at 20-25°C for 10-12 hours. The resulting powder-type crystals were filtered. The filtered crystals were dried under vacuum at 70-80°C for 24 hours to obtain 240 g of powder-type particles of crystalline Form I (yield 92%).

### Comparative Example 2: Preparation of powder-type particles of clopidogrel bisulfate (crystalline Form I)

200 g of clopidogrel free base obtained in Example <1-1> was dissolved in 2,000 mL of 2-propanol, followed by maintaining at 26 to 28°C. Then, a solution of 62.3 g of sulfuric acid (98%) in 400 mL of cyclohexane was added dropwise thereto for 3 hours. The mixture was vigorously stirred at 18-20°C for 6 hours. The resulting powder-type crystals were filtered. The filtered crystals were dried under vacuum at 70-80°C for 24 hours to obtain 250 g of powder-type particles of crystalline Form I (yield 94%).

### Test Example 1: Measurement of particle size distribution

The particles obtained in Example 1 and Comparative Examples 1 and 2 were analyzed for particle size distribution using a particle size analyzer (Mastersizer 2000^{®}, Malvern) based on laser diffraction. The samples were injected at a pressure of 1 bar with a dry module (Scirocco 2000®, Malvern). Each particle was measured for particle size (d0.1), particle size (d0.5) and particle size (d0.9). The results are shown in Table 1 below.

**[Table 1]**

| Item | Example 1 | Comp. Example 1 | Comp. Example 2 |
|---|---|---|---|
| d(0.1) | 52.536 µm | 1.052 µm | 8.475 µm |
| d(0.5) | 74.567 µm | 6.055 µm | 85.770 µm |
| d(0.9) | 106.074 µm | 13.724 µm | 168.393 µm |

### Test Example 2: Stability Comparison between direct compression and wet granulation

The spherical particles of clopidogrel bisulfate (crystalline Form I) obtained in Example 1 was compressed into a tablet by using direct compression and wet granulation, respectively, and during above process, discoloration or increased impurities were compared. The prepared tablets were placed into petri-dishes and stored under an accelerated test condition (temperature: 40±2°C, relative humidity: 75±5%) for content and purity test in accordance with U.S Pharmacopoeia for clopidogrel bisulfate tablet.

Specifically, the tablet according to direct compression was prepared using the composition and the method described in Example 2, and the tablet according to wet granulation was prepared using the composition described in Example 2. The spherical particles of clopidogrel bisulfate prepared in Example 1, lactose hydrate and low-substituted hydroxypropylcellulose were mixed, and an aqueous solution of 10% (w/v) hydroxypropylcellulose as a binder solution was added thereto for granulation. The granules were assembles at 60°C for 12 hours, and colloidal silicon dioxide and sodium stearyl fumarate were added thereto, followed by compressing the granules using a rotary tablet machine.

Table 2 below lists ingredients used in direct compression and wet granulation, and Table 3 shows the content of clopidogrel and related substances in the tablets.

**[Table 2]**

| Ingredient | Direct compression | Wet granulation |
|---|---|---|
| | One tablet (mg) | One tablet (mg) |
| Clopidogrel bisulfate (crystalline Form I) - Example 1 | 97.875 | 97.875 |
| Lactose hydrate (SuperTab 11SD^{®}) | 120.625 | - |
| Lactose hydrate (Pharmatose 200M^{®}) | - | 116.625 |
| Low-substituted hydroxypropylcellulose(LH-11^{®}) | 30.0 | 30.0 |
| Hydroxypropylcellulose (HPC-L) | - | 4.0 |
| Colloidal silicon dioxide | 2.5 | 2.5 |
| Sodium stearyl fumarate | 7.0 | 7.0 |
| Total weight of tablet | 258.0 | 258.0 |

**[Table 3]**

| Item | Direct compression | | Wet granulation | |
|---|---|---|---|---|
| | Initial | 2 weeks | Initial | 2 weeks |
| Appearance | White tablet | Yellowish tablet | Yellowish tablet | Yellow tablet with brown spots |
| Related substance A | Not detected | 0.13% | 0.36% | 0.96% |
| Related substance C | 0.03% | 0.09% | 0.07% | 0.20% |
| Individual Unidentified related substance | Maximum 0.11% | Maximum 0.50% | Maximum 0.16% | Maximum 0.93% |
| Total related substances | 0.16% | 1.32% | 0.65% | 2.99% |
| content | 99.73% | 98.51% | 99.47% | 96.66% |

As seen from above results, the tablet prepared by wet granulation exhibited an increased discoloration and impurities during the process, and the increase was significant over the course of time, compared to the tablet prepared by direct compression. Therefore, it was found that direct compression is suitable for clopidogrel bisulfate in terms of physicochemical stability.

### Examples 2 to 4: Preparation of tablets using spherical particles of clopidogrel bisulfate

In accordance with the compositions shown in Table 4 below, tablets were prepared using the spherical particles of clopidogrel bisulfate Form I prepared in Example 1.

In addition to clopidogrel bisulfate, lactose hydrate (SuperTab 11SD^{®}), D-mannitol (Pearlitol 100SD^{®}) and silicified microcrystalline cellulose (Prosolv HD90^{®}) as excipients for direct compression, low-substituted hydroxypropylcellulose (LH-11^{®}) as a disintegrating agent, colloidal silicon dioxide (Aerosil 200^{®}) as a fludizing agent and sodium stearyl fumarate (Pruv^{®}) as a lubricating agent were sieved through a #30 mesh, mixed, and compressed into a tablet by direct compression using a rotary tablet machine. Then, the tablet was coated with a coating solution of a film-coating agent, Opadry pink, in a mixture of ethanol and purified water, followed by drying.

**[Table 4]**

| Ingredient | Example 2 | Example 3 | Example 4 |
|---|---|---|---|
| | One tablet (mg) | One tablet (mg) | One tablet (mg) |
| Clopidogrel bisulfate (crystalline Form I) - Example 1 | 97.875 | 97.875 | 97.875 |
| Lactose hydrate | 120.625 | - | - |
| D-mannitol | - | 120.625 | - |
| Silicified Microcrystalline cellulose | - | - | 120.625 |
| Low-substituted hydroxypropylcellulose | 30.0 | 30.0 | 30.0 |
| Colloidal silicon dioxide | 2.5 | 2.5 | 2.5 |
| Sodium stearyl fumarate | 7.0 | 7.0 | 7.0 |
| Total weight of tablet | 258.0 | 258.0 | 258.0 |
| Opadry pink | 10.0 | 10.0 | 10.0 |
| Total weight of tablet | 268.0 | 268.0 | 268.0 |

### Comparative Examples 3-8: Preparation of tablets using clopidogrel bisulfate powder

In accordance with the compositions shown in Table 5 below, tablets were prepared using a powder of clopidogrel bisulfate (Crystalline Form I) obtained in Comparative Examples 1 and 2.

In addition to each clopidogrel bisulfate, lactose hydrate (SuperTab 11SD^{®}), D-mannitol (Pearlitol 100SD^{®}) and silicified microcrystalline cellulose (Prosolv HD90^{®}) as excipients for direct compression, low-substituted hydroxypropylcellulose (LH-11^{®}) as a disintegrating agent, colloidal silicon dioxide (Aerosil 200^{®}) as a fludizing agent and sodium stearyl fumarate (Pruv^{®}) as a lubricating agent were sieved through a #30 mesh, mixed, and compressed into a tablet by direct compression using a rotary tablet machine.

**[Table 5]**

| Ingredient | Comp. Ex. 3 | Comp. Ex. 4 | Comp. Ex. 5 | Comp. Ex. 6 | Comp. Ex. 7 | Comp. Ex. 8 |
|---|---|---|---|---|---|---|
| | One tablet (mg) | One tablet (mg) | One tablet (mg) | One tablet (mg) | One tablet (mg) | One tablet (mg) |
| Clopidogrel bisulfate (crystalline Form I) | 97.875 | 97.875 | 97.875 | - | - | - |
| Clopidogrel bisulfate (crystalline Form II) | - | - | - | 97.875 | 97.875 | 97.875 |
| Lactose hydrate | 120.625 | - | - | 120.625 | - | - |
| D-mannitol | - | 120.625 | - | - | 120.625 | - |
| Silicified microcrystalline cellulose | - | - | 120.625 | - | - | 120.625 |
| Low-substituted hydroxypropylcellulose | 30.0 | 30.0 | 30.0 | 30.0 | 30.0 | 30.0 |
| Colloidal silicon dioxide | 2.5 | 2.5 | 2.5 | 2.5 | 2.5 | 2.5 |
| Sodium stearyl fumarate | 7.0 | 7.0 | 7.0 | 7.0 | 7.0 | 7.0 |
| Total weight of tablet | 258.0 | 258.0 | 258.0 | 258.0 | 258.0 | 258.0 |

### Test Example 2: Comparison of compressibility between spherical particles and powders of clopidogrel bisulfate

While compressing the compositions described in Examples 2 to 4 and Comparative Examples 3 to 8 into tablets, the occurrence of problems in compressing tablets such as sticking was monitored. In addition, the thirty compressed tablets were tested for weight variation in accordance with general tests of Korean Pharmacopoeia. Furthermore, the tablets were measured for their hardness using a hardness analyzer. The measurement results are shown in Table 6.

**[Table 6]**

| Item | Sticking | Acceptance value in weight variation test (%) | Hardness(kp) |
|---|---|---|---|
| Example 2 | x | 2.4 % | 10 ~ 12kp |
| Example 3 | x | 2.8 % | 10 ~ 12kp |
| Example 4 | x | 2.6 % | 11 ~ 13kp |
| Comparative Example 3 | o | 17.7% | 3 ~ 5kp |
| Comparative Example 4 | o | 18.3% | 2 ~ 4kp |
| Comparative Example 5 | o | 17.4% | 3 ~ 5kp |
| Comparative Example 6 | o | 15.2% | 4 ~ 6kp |
| Comparative Example 7 | o | 15.5% | 4 ~ 6kp |
| Comparative Example 8 | o | 15.7% | 5 ~ 7kp |

As shown in Table above, for Comparative Example 3 to 8 comprising a clopidogrel bisulfate powder, the mixture was not smoothly supplied into a die due to poor flowability and the acceptance value exceeded the acceptance criteria (15%). Further, the hardness of the tablets was significantly weak and serious sticking occurred, making it impossible for tableting. On the contrary, the mixture containing clopidogrel bisulfate spherical particles in accordance with Examples 2 to 4 exhibited good flowability, uniform weight, and superior strength, and no problem in compressing tablets.

### Test Example 3: Stability test under severe conditions

The tablet of the present invention prepared in Example 2 and the commercially available Plavix tablet (Plavix^{®}, Sanofi-Aventis) were placed into petri-dishes and evaluated for stability under an accelerated test condition (temperature: 40±2°C, relative humidity: 75±5%) by measuring content and purity in accordance with of U.S. Pharmacopeia for clopidogrel bisulfate tablet. The measurement results are shown in Table 7.

**[Table 7]**

| Item | Example 2 | | Plavix Tab | |
|---|---|---|---|---|
| | Initial | Two weeks | Initial | Two weeks |
| Appearance | Pink tablet | Pink tablet | Pink tablet | Discoloration |
| Related substance A | Not detected | 0.10% | 0.05% | 0.72% |
| Related substance C | 0.04% | 0.07% | 0.07% | 0.95% |
| Individual unidentified related substance | Maximum 0.11% | Maximum 0.36% | Maximum 0.04% | Maximum 0.87% |
| Total related substances | 0.17% | 0.95% | 0.20% | 3.05% |
| Contents | 99.65% | 98.93% | 99.47% | 95.26% |

As seen from Table 7, the example 2 comprising spherical particles of clopidogrel bisulfate has no change in appearance, and a significantly slow increase in impurities and content, compared to commercially available Plavix tablet, indicating improved physicochemical stability of the tablet according to the present invention.

### Test Example 4: Stability Test under accelerated conditions

The tablet of the present invention prepared in Example 2 was packaged with silica gel in a polyethylene bottle, and stored under acceleration conditions of 40°C /RH 75%. The tablet was measured for stability six months later in accordance with U.S. Pharmacopeia for Clopidogrel Bisulfate Tablet. The measurement results are shown in Table 8.

**[Table 8]**

| Item | Criteria | Initial | 3 months | 6 months |
|---|---|---|---|---|
| Appearance | Round-shape of pink, film-coated tablet | Appropriate | Appropriate | Appropriate |
| Dissolution | 30 min 80% (Q) | 99.38∼101.38 % | 98.5∼100.36 % | 98.72∼101.12 % |
| Purity | Related substance A: 1.2% or less; | Not detected | 0.03% | 0.09% |
| | Related substance C 1.5% or less; | 0.04% | 0.06% | 0.08% |
| | Unknown compounds: 0.2% or less; | Max 0.11% | Max 0.13% | Max 0.14% |
| | Total related substances: 2.5% or less | 0.17% | 0.27% | 0.39% |
| Content | 95.0~105.0% | 99.65% | 99.16% | 98.76% |

As shown in Table 8, the tablet according to the present invention exhibited good stability which meets the criteria of all items including appearance, dissolution, purity and content.

## Claims

1. Spherical particles of clopidogrel bisulfate having a particle size (d0.1) of 30 µm or more and a particle size (d0.5) of 50 to 200 µm.

2. The spherical particles of claim 1, wherein the clopidogrel bisulfate is crystalline form I, crystalline form II, or a mixture thereof.

3. A pharmaceutical composition comprising the spherical particles of clopidogrel bisulfate of claim 1; pharmaceutically acceptable excipient for direct compression; low-substituted hydroxypropylcellulose as a disintegrating agent; colloidal silicon dioxide as a fludizing agent; and sodium stearyl fumarate as a lubricating agent.

4. The pharmaceutical composition of claim 3, wherein the pharmaceutical composition does not contain a binding agent.

5. A tablet prepared from the pharmaceutical composition of claim 3 by direct compression.

6. A method for preparing a tablet containing spherical particles of clopidogrel bisulfate, comprising the steps of:
1) mixing the spherical particles of clopidogrel bisulfate of claim 1; a pharmaceutically acceptable excipient for direct compression; low-substituted hydroxypropylcellulose as a disintegrating agent; colloidal silicon dioxide as a fludizing agent; and sodium stearyl fumarate as a lubricating agent; and
2) subjecting the mixture to direct compression without adding a binding agent, to form a tablet.
